# EUROPEAN PATENT SPECIFICATION

(11) **EP 3 089 766 B1**
(45) Date of publication and mention of the grant of the patent: **06.03.2019**
(21) Application number: 15703112.1
(22) Date of filing: 01.01.2015
(51) Int. Cl.: A61L 24/00

(54) **OPHTHALMIC VISCOSURGICAL DEVICE**
OPHTHALMISCHE VISKOCHIRURGISCHE VORRICHTUNG
DISPOSITIF VISCOCHIRURGICAL OPHTALMIQUE

(30) Priority: 02.01.2014 US 201461922913 P
(43) Date of publication of application: 09.11.2016
(73) Proprietor: Yissum Research Development Company of the Hebrew University of Jerusalem Ltd., 91390 Jerusalem (IL); Shaare Zedek Scientific Ltd, Jerusalem 91031 (IL)
(72) Inventor: COHN, Daniel, 9371219 Jerusalem (IL); ROZENMAN, Yaacov, 9371420 Jerusalem (IL); MALAL, Ram, 9622806 Jerusalem (IL); DRABKIN, Yelena, 7171501 Modiin (IL); ELYASHIV, Ariel, Neve Daniel 9090900 (IL)
(74) Representative: Boult Wade Tennant LLP
(86) International application number: PCT/IL2015/050011
(87) International publication number: WO 2015/102000

(56) References cited:
- WO-A1-2005/082333
- WO-A1-2005/097225
- WO-A2-02/36096
- US-A- 4 851 513

## Description

### FIELD OF THE INVENTION

The invention generally relates to materials and compositions for use as ophthalmic viscosurgical devices (OVDs).

### BACKGROUND OF THE INVENTION

There is a wide variety of materials which are foreign to the human body and which are used in direct contact with its organs, tissues and fluids. These materials are called biomaterials; and among them, polymers play a pivotal role. In some instances, it is detrimental or even dangerous for a device to remain at the location where it performs its function, once completed. Such devices need, therefore, to be removed from the site following an external action or stimulus.

A cataract is a clouding of a region of or the entire eye lens, resulting in blurred or distorted vision, which generally occurs in the elderly. Typically, cataract surgery involves removal of the cataractous lens from the capsular bag and its replacement with an artificial intraocular lens (IOL). This procedure involves two incisions; one through the sclera or cornea into the anterior chamber of the patient's eye and another into the capsular bag.

Cataract surgery is performed using a phacoemulsification technique that utilizes a high-intensity ultrasound energy for the fragmentation and emulsification of the cataractous lens. Once the cloudy natural lens has been removed, an IOL is inserted into the capsular bag and deployed therein. The overall procedure is potentially traumatic to the tissue surrounding the anterior chamber. To reduce the amount of trauma to the patient's eye, an Ophthalmic Viscosurgical Device (OVD) is injected into the anterior chamber and into the capsular bag. An OVD provides a physical barrier and creates space, balances pressure in the anterior and posterior segments of the eye, stabilizes tissue and, most importantly, protects corneal endothelial cells. Cataract removal is the most frequently conducted surgical procedure in the world (around 20 million performed every year worldwide).

The corneal endothelium consists of a monolayer of cells present on the posterior corneal surface and, since they have no regenerative ability, endothelial cells damaged during surgery are irreversibly lost. The normal thickness and transparency of the cornea are maintained by the diffusion barrier provided by a contiguous and healthy endothelium and by the active fluid pump function performed by the corneal endothelial cells.

It is, therefore, of the utmost importance to protect the integrity of the corneal endothelium during cataract surgery. Intraocular manipulation during cataract surgery, and especially the phacoemulsification of the cataractous lens, may significantly damage the endothelium. During the high energy phacoemulsification stage, apart from the direct harmful effect of the ultrasonic energy, fragments of the cataractous lens are formed and are forcefully propelled towards the endothelium surface, damaging the endothelial cells.

OVDs are also used during Glaucoma surgery. There are well known surgical techniques to treat glaucoma. The most widely used is the trabeculectomy, which involves removing a portion of the scleral tissue that includes part of the trabecular meshwork and Schlemm's canal.

Another way to treat glaucoma is to surgically implant a drainage device in the eye. The drainage device functions to allow aqueous humor to drain from the anterior chamber and thereby reduce the intraocular pressure. The drainage device is usually implanted using an invasive surgical procedure. Pursuant to such a procedure, a flap is surgically cut in the sclera; he flap is folded back to form a small pocket and the drainage device is inserted into the eye through the flap. This procedure can be quite problematic as the implants are large and can result in various adverse events such as infections, erosions, and scarring, leading to the need to re-operate.

Since the introduction of sodium hyaluronate in ophthalmic surgery in the early 1970s, viscoelastic materials have assumed a major role in anterior segment surgery. New viscoelastic materials have been developed primarily to improve protection of corneal endothelial cells during phacoemulsification. Currently, there are two main types of viscoelastic materials for enhanced protection of the corneal endothelium: (1) high-viscosity, cohesive materials (e.g., sodium hyaluronate), which are form-maintaining, and (2) low viscosity, dispersive materials such as sodium chondroitin sulfate 4%-sodium hyaluronate 3% such as Viscoat® and hydroxypropyl methylcellulose 2% such as Ocucoat®), which adhere to the corneal endothelium, providing a protective layer throughout the surgical procedure. Such materials are often used in patients with a low pre-operative endothelial cell count, shallow anterior chambers and hard nucleus. Each of these two classes of OVDs has advantages but also inherent drawbacks.

While the low viscosity OVDs are easy to inject, they supply poor protection to the corneal endothelium. On the other hand, high viscosity OVDs provide better protection to endothelial cells, but they are difficult to inject as well as to remove. Further it is consensually accepted that the OVDs presently in clinical use, do not provide sufficient endothelial protection, this being especially pronounced in cases where a hard nucleus, shallow chamber and poor endothelium are present.

Almeida H, et al [**1**] reported on temperature and pH stimuli-responsive polymers and their applications in controlled and self-regulated drug delivery.

Kushwaha SK, et al [**2**] reported on stimuli sensitive hydrogels for ophthalmic drug delivery.

WO02/36096 A discloses improved viscous or viscoelastic agents having a variable or transitional viscosity such that it will become substantially less viscous after surgery.

### REFERENCES

[**1**] Almeida H, et al., Journal of Applied Pharmaceutical Science. 07/2012; 2(6):1-10.
[**2**] Kushwaha SK, et al., Int J Pharm Investig. 2012; 2(2):54-60.

### SUMMARY OF THE INVENTION

A difficulty that accompanies various types of ophthalmic surgical maneuvers (e.g. cataract and glaucoma treatemnt) is to maintain the normal ('uncollapsed') configuration of the anterior segment of the eye. Damage to the eye's anterior chamber may result in irreversible damage to the vital corneal endothelial cells resulting in permanent corneal edema and consequently blurred vision.

Surgical benefits of using ophthalmic viscosurgical device (OVD) during ophthalmic surgical maneuvers include maintenance of the anterior chamber, protection of the corneal endothelium, and facilitation of intraocular lens implantation. However, while cataract and various other surgical and laser eye procedures which employ endothelium protection by using an OVD are considered common practice these procedures carry a risk of OVD being left in the eye post surgery resulting in unwanted and sometime hazardous effects such as post surgery intra ocular pressure (IOP) spikes. For example, such IOP spikes from remaining OVD material may endanger patients with glaucoma.

Additionally, as noted above, many of the OVDs known to date are either difficult to handle during surgery or eye treatment or supply poor protection to the corneal endothelium.

To prevent such difficulties and risk of post surgery OVD leftovers in the anterior segment of the eye, to facilitate and improve OVD function, which can also be easily aspirated out from the eye after completion of surgery, the inventors have developed a novel class of ophthalmic viscosurgical devices (OVDs) which have superior advantages in comparison to known OVDs, and further avoid many of the drawbacks known for available and known OVDs.

The present invention relates to an ophthalmic viscosurgical device (OVD) characterized by comprising a reverse thermo-sensitive material.

The scope of the invention is defined by the claims. Any references in the description to methods of treatment refer to the compounds, pharmaceutical compositions and medicaments of the present invention for use in a method for treatment of the human (or animal) body by therapy (or for diagnosis). The OVDs of the invention are characterized as having one or more of the following features:
- non-toxic to ocular tissues, mainly to the endothelial cell layer;
- easy to insert and remove through a small bore needle;
- remains clear throughout the surgery;
- remains as gel throughout the surgery;
- remains in place throughout surgery, does not leak;
- does not damage the endothelium, mechanically;
- Does not cause inflammation/uveitis; and/or
- does not cause pressure/glaucoma.

Since lower viscosity OVDs often leak after less than 24 hours post injection, the OVD of the present invention is a suitable substitute as it maintains (e.g. after stimuli and transition into a second physical state) a highly viscous constitution and can therefore fill the anterior chamber for the required time period.

The OVDs of the present invention may be utilized in a variety of ophthalmic procedures and have been shown to be capable of maintaining the intactness of the anterior segment of the eye during eye surgery. Thus, a general method utilizing OVDs according to the invention permits their administration to the eye, and their extraction from the eye following a surgical or other treatment of the eye region.

As used herein, an "***ophthalmic viscosurgical device***" (OVD) (interchangeable herein with *OVD material*) generally refers to at least one material which is compatible (e.g. isotonic, pH balanced, based on an aqueous solution) for use in eye surgery or eye treatment (e.g. suitable for administration to the mammalian eye). The ability of a OVD of the invention to "***maintain the intactness of the anterior segment of the eye***" makes it possible to achieve at least one beneficial effect and to enable a stable and safe yet flexible environment for eye surgery or eye treatment, accompanied with better visual recovery following the introduction (e.g. by injection) of the OVD into the eye. Thus, maintaining the intactness of the anterior segment of the eye may encompass all or any combination of the following desirable outcomes that follow the use of an OVD of the present invention: protecting cornea, iris and other eye structure during phacoemulsification; segregating damaged iris from phacoemulsification; protecting (e.g. by creating a protective barrier) the corneal endothelium to prevent endothelial cell loss; maintaining of the intactness of the anterior chamber after phaco-trabecalectomy or trabecalectomy alone; helping reduce free radicals, creating and maintaining space within the anterior chamber, aiding in manipulation of eye tissues during surgery, maintaining clarity throughout the surgical field.

As a person of skill in the art would understand, the anterior segment of the eye refers to both the anterior chamber and to the capsular bag of the eye.

In order to permit easy of handling, the OVDs of the invention are constructed from materials which are responsive to one or more pre-selected stimulus and which change their physical state in response to such a stimulus. Thus, a method utilizing a OVD according to the invention may comprise administering into an eye of a subject a OVD at a first physical state measured under a stimulus outside the eye, e.g., the first physical state may be a lower viscosity; and affecting a second stimulus while the OVD is in the eye to transition to a second desirable physical state. In some cases, the transition to the second physical state may be spontaneous upon relieve of the stimulus.

Thus, the invention contemplates a method comprising:
- administering into an eye of a subject a OVD at a first physical state measured (and formed) under a stimulus outside the eye, e.g., the first physical state may be a lower viscosity; and
- affecting a stimulus to the OVD in the eye to transition to a second physical state, e.g., a state which is different from the first physical state and which for example be a solid state or semi-solid, having higher viscosity.

In some embodiments, the first stimulus to form the first physical state is applied to the OVD while in the second physical state, to transform the OVD into the first physical state and once the stimulus is arrested, the OVD transforms back to its original second physical state. For example, in cases where the OVD is available for use in a solid or semi-solid form, for easy of delivery, it may be stimulated to adopt a more fluid, liquid physical form. Once delivered to the eye, the liquid OVD may spontaneously transform back to the solid or semi-solid form. Thus, the liquid form may be regarded as the first physical form and the solid or semi-solid form may be regarded as the second physical form.

Once the OVD is placed in the eye, the surgical procedure or any treatment of the eye may begin. Thus, a method involving the OVD may further comprise:
- performing the ophthalmic procedure and once the procedure has been completed, applying to the OVD in the eye a further stimulus (which may or may not be the same as the previously applied stimulus) to thereby revert the OVD to its first physical state or to a third physical state which is different from the first and second states and which permits the OVD's removal (e.g., the first or third physical state may be liquid state or semi-liquid state of a lower viscosity); and
- removing the OVD in its first or third physical state from the eye.

The OVDs used in methods and compositions of the invention are typically "smart" polymers and/or copolymers that are selected from an advanced class of materials tailored to display substantial property changes, in response to chemical, physical or biological stimuli, such as and without limitation, temperature, pH, biochemical agents, mechanical stresses, magnetic and electrical fields.

The property changes are typically in the physical state of the OVD, namely in the ability to affect transition from a liquid or semi-liquid state to a gel or solid or semi-solid state. This ability to transform between physical states, while maintaining the ability to maintain the intactness of the anterior segment of the eye, as defined herein, renders the OVDs of the invention superior to OVDs known in the art.

The delivery of the OVD to the anterior segment thus requires that the OVD is presented in a form or a physical state which is user-friendly and easy to achieve. As used herein, the OVD is deliverable into the eye in a "*f**irst physical state***", namely in a state of low to medium viscosity, being not higher than (or lower than, or up to) about 100,000 Pa.

The viscosity of the OVD of the present invention is defined herein as G' [Pa] (G' being the storage modulus of the solution) and is generally measured using a Thermo-HAAKE RO1 rheometer employing a plate-plate configuration and conducting an oscillation vs temperature test with a PP35 sensor, at a constant shear stress of 5Pa and a constant frequency of 10Hz. At such a viscosity, the OVD may be introduced (e.g. by injection) into the anterior chamber of the eye. The low to medium viscosity state is advantageous in that it enables administration of the OVD using a small syringe and cannula which is often difficult when trying to introduce OVDs of higher viscosities.

In some embodiments, the first physical state is defined by a viscosity not higher than about 100,000Pa.

In some embodiments, the viscosity of the first physical state is between 0.01 and between about 90,000Pa or between 0.01 and between about 80,000Pa, or between 0.01 and between about 70,000Pa or between 0.01 and between about 60,000Pa or between 0.01 and between about 50,000Pa or between 0.01 and between about 40,000Pa or between 0.01 and between about 30,000Pa or between 0.01 and between about 20,000Pa.

In some embodiments, the first physical state is defined by a viscosity of between about 20,000Pa and between about 100,000Pa.

Once in the eye, the OVD should transform to a more solid state, referred to herein as the "***second physical state***", which generally refers to a medium to high viscosity state being at least twice the viscosity of the first physical state. At such a viscosity, the ophthalmic procedure may be carried out in a safe and fast manner. Thus, in accordance with the present invention, the second physical state of the OVD allows for retention of the OVD in the eye to thereby provide adequate space within the anterior chamber to allow manipulation of intraocular tissues and/or to coat and protect cellular structures for a desirable time period, while maintaining space and easing mechanical operation depending on the specific requirement governed by the type of ophthalmic procedure performed in the eye (e.g. retention in the anterior chamber to safeguard endothelial cells throughout the entire phacoemulsification process). The second physical state of the OVD enables to obtain a viscous stationary OVD which viscosity does not substantially change with shear rate.

In some embodiments, the second physical state is defined by a viscosity at least twice as high as the viscosity of the first physical state. In other words, the viscosity of the second physical state is equal or higher than about 200,000Pa.

In some embodiments, the viscosity of the second physical state is between about 200,000 and between about 400,000Pa or between about 200,000 and between about 600,000Pa or between about 200,000 and between about 1000,000Pa or between about 200,000 and between about 2000,000Pa or between about 200,000 and between about 3000,000Pa or between about 200,000 and between about 4000,000Pa or between about 200,000 and between about 6000,000Pa or between about 200,000 and between about 8000,000Pa or between about 200,000 and between about 10,000,000Pa.

In some embodiments, the viscosity of the second physical state is higher than the viscosity of the first physical state. In some embodiments, the viscosity of the second physical state is 1.2 times the viscosity of the first physical state, or is 1.4, 1.6, 1.8, 2, 2.2, 2.4, 2.6, 2.8, 3, 3.2, 3.3, 3.4, 3.5, 3.6, 3.7, 3.8, 4, 5, 6, 7, 8, 9, or 10 times the viscosity of the first physical state.

For the purpose of removing the OVD from the anterior segment of the eye, the physical state of the OVD should be revered to the above indicated first physical state or to a different state which is different from the first and second physical states, but which similarly to the first state endows a viscosity which permits the removal of the OVD. Thus, as used herein, the OVD is removed from the eye in a "***first or third physical state***", namely in a state of low to medium viscosity, having a viscosity not higher than about 100,000Pa.

In some embodiments, the viscosity of the third physical state is defined by a viscosity not higher than about 100,000Pa.

In some embodiments, the viscosity of the third physical state is between 0.01 and between about 90,000Pa or between 0.01 and between about 80,000Pa, or between 0.01 and between about 70,000Pa or between 0.01 and between about 60,000Pa or between 0.01 and between about 50,000Pa or between 0.01 and between about 40,000Pa or between 0.01 and between about 30,000Pa or between 0.01 and between about 20,000Pa.

In some embodiments, the third physical state is defined by a viscosity of between about 20,000Pa and between about 100,000Pa.

The "***ophthalmic procedure***" may be any surgical or none surgical task which is used to benefit a mammalian eye (human or animal eye) or any part thereof and/or to protect the eye from trauma (e.g., as a viscoelastic for iris and capsule protection, as a space filler/maintainer). Some non limiting examples of ophthalmic procedures include phacoemulsification surgery (including during rhexis as a phacoemulsificator), corneal transplant, anterior chamber intraocular lens implantation, phaco-trabecalectomy or trabecalectomy alone, cataract surgery (including intra and extra -capsular cataract surgery), glaucoma surgery, corrective laser eye surgery, keratoplasty, canaloplasty, refractive surgery, corneal surgery (including a corneal "jack" device), vitreo-retinal surgery, eye muscle surgery, oculoplastic surgery (e.g., eyelid surgery, orbital surgery), intra ocular lens implantation, graft (e.g., corneal) surgery, retinal procedures, and ocular trauma treatments.

Typically, the OVD of the invention is not employed in accordance with embodiments of the invention for drug delivery. In some embodiments, where the OVD comprises one or more therapeutic agent, it is only for use in conjunction with method steps of the invention.

In some embodiments, the OVD is free of any therapeutic agents. In further embodiments, the OVD consists of a polymeric material, as defined herein, being the OVD material.

The "***stimulus***" may be any chemical, physical or biological stimuli as further described herein, e.g., temperature (including room temperature), pH, ionic strength, biochemical agents, mechanical stresses, and application of magnetic or electrical fields. As a person of skill in the art would understand, the choice of stimuli and strength thereof depends on various parameters such as the intended ophthalmic procedure to be performed in the eye and the nature of the OVD. The stimulus is always selected to have no negative effect on the eye or the eye region or on the ophthalmic procedure.

The OVD is formed upon administration of the material, in its low viscosity form, into the (e.g., anterior chamber of the) eye of the animal (human or non-human mammal). The conversion of the material to a form having a higher viscosity or a semi-solid or solid consistency (second physical state) occurs in the site where the conversion to a higher viscosity is required, namely in the eye. The conversion to a higher viscosity form may be achieved by a stimulus that is a natural condition or parameter of the eye (e.g. eye temperature) and may be maintained during the procedure or through external means, i.e., stimulus, which is applied to the material (such as ionic strength or electrical current).

The stimulus used in a process for forming the first physical state OVD or for removing OVD from the eye post surgery, namely at the first or third physical states, may or may not be the same. In fact, in some embodiments, the stimulus used to decrease or maintain the viscosity of the OVD may be different from the stimulus used to increase the OVD viscosity.

In some embodiments, the OVD material has one viscosity under a stimulus and a second higher viscosity under a second or different stimulus. In other embodiments, the viscosity may be modulated (increased or decreased) by either reapplying one of the employed stimuli or by applying a new and different stimulus.

The stimuli used in the different stages of the process to affect the first, second and/or third physical states, may be of the same type (e.g., different temperatures) or of different types (one temperature and one electrical current or pH) and are typically selected from temperature, pH, presence of a biochemical agent, ionic strength, mechanical stress, presence of an electrical field, presence of a magnetic field and combinations thereof.

In accordance with some embodiments, the stimulus is temperature.

In some embodiments, the stimulus is applied continuously; for example the OVD is maintained at a certain temperature throughout the entire ophthalmic procedure. In further embodiments, the stimulus is transient or is applied over a period of time sufficient to transform all or a predetermined portion of the OVD into the desired physical state.

In accordance with other embodiments, the material has a lower viscosity at a temperature below body temperature and a higher viscosity at a body temperature (e.g., the temperature of the eye).

As uses herein "***administering***" generally refers to any method of administration of the OVD to the eye which includes, but is not limited to, injection. Other alternative methods of administration include eye drops.

As used herein "***removing***" of the OVD material generally refers to removal of the OVD material by aspiration (e.g. using a cannula), irrigation or by any known means commonly available to the practitioner.

The OVDs of the invention may further be used in a method for maintaining the intactness of the anterior segment of the eye during eye surgery, as described herein.

The invention further concerns a use of at least one material for the preparation of a OVD, said material, as detailed herein, changes its viscosity upon application of pre-defined stimuli.

In still another one of its aspects, the present invention provides a method for forming an Ophthalmic Viscosurgical Device (OVD) in the anterior chamber and/or in the capsular bag of a mammalian eye, the method comprising:
- administering an OVD material into the eye, the OVD material being at a first physical state outside the eye; and
- affecting a stimulus to the OVD material in the eye to transition to a second physical state (e.g., to a solid or semi solid viscosity).

Once the OVD is placed, the surgical procedure may begin, and thus the process may further comprise:
- performing the ophthalmic procedure;
- once the procedure has been completed, applying to the material in the eye a stimulus to revert to a first physical state or to a third physical state; and
- removing the material from the eye.

As may be understood by a person versed in the art, the OVD material at its first physical state, at its second physical state or in the time period defining the transition between the two states is protective to ocular cellular structures and eases mechanical operation during the herein described ophthalmic procedure.

In some embodiments, the OVD is used to coat surgical instruments and intraocular lens (IOL).

In some embodiments, the OVD is for coating and protecting ocular structures.

In some embodiments, the OVD is for maintaining anterior chamber space in the eye.

In some embodiments, the OVD is for protecting cornea iris and other eye structure during phacoemulsification.

In some embodiments, the OVD is for segregating damaged iris from phacoemulsification.

In some embodiments, the OVD is for creating a stable yet flexible environment for eye surgery.

In some embodiments, the OVD is for use during corneal transplant.

In some embodiments, the OVD is for use during anterior chamber intraocular lens implantation.

In some embodiments, the OVD is for use in maintenance of the anterior chamber after phaco-trabecalectomy or trabecalectomy alone.

The material from which the OVD is formed may comprise a single compound, or a mixture of compounds or complex of several compounds. The material may be a polymeric or non-polymeric material, as described below. Whatever material is chosen for an OVD of the invention, the material or material combination should be capable of changing its viscosity upon application of pre-defined stimuli.

In some embodiments, where the material is a combination (mixture) of two or more compounds, each of the components of the material may be administered (e.g., by injection) separately (e.g., simultaneously or sequentially) or as a combination.

In some embodiments, the material employed in accordance with the invention is a thermo-sensitive polymer; namely, the polymer being capable of undergoing a chemical, mechanical, physical and/or biological change under small temperature differentials. The change is based on different mechanisms such as ionization and entropy gain due to release of water molecules, among others (Alexandridis and Hatton, Colloids and Surfaces A, 96, 1 (1995)).

The thermo-sensitive gels employed in accordance with the invention can be classified into two categories: (a) positive-sensitive hydrogels- having upper critical solution temperatures (UCST); these hydrogels contract upon cooling below the UCST, and (b) negative-sensitive hydrogels- having lower critical solution temperatures (LCST); these contract upon heating above the LCST temperature.

In some embodiments, the gels are LCST displaying systems.

In other embodiments, the polymer employed in accordance with the invention is selected amongst those having Reverse Thermal Gelation (RTG) properties. The reverse thermo-responsive phenomenon is usually known as Reverse Thermal Gelation (RTG) and it constitutes one of the most promising strategies for the development of injectable systems. The water solutions of these materials display low viscosities at low temperatures (below, at or above ambient temperature), and exhibit a sharp viscosity increase as the temperature rises within a very narrow temperature interval, producing a semi-solid gel once they reach the body temperature. In the case of the present invention, the thermal transition has to be below the temperature of the eye tissues relevant to OVDs' performance.

The OVDs of the invention may comprise any thermo-responsive material, polymeric or not, that displays the temperature dependent viscosity differential required. For the sake of simplicity and conciseness, the rest of this document will confine itself to polymeric materials but this should not be construed in any way or fashion as limiting the present invention only to polymers. In some embodiments, the concentration of the polymer in the OVD is adjusted to render the OVD with a desired liquid-gel transition.

The thermo-responsive materials are in some embodiments polymeric, and among them, without limitation, polymers such as poly(ethylene oxide)-poly(propylene oxide)-poly(ethylene oxide) (PEO-PPO-PEO) triblocks, random or alternating reverse thermo-responsive PEO-PPO block copolymers, tetrafunctional block polymers of polyoxyethylene and polyoxypropylene condensed with ethylenediamine, N-alkyl substituted acrylamides (preferably poly-N-isopropyl acrylamide [PNIPAAm], cellulose derivatives, selected from a group consisting of hydroxypropyl methylcellulose and hydroxypropyl cellulose, alternating or random, and various amphiphilic polymers such as poly(ethylene oxide)-polylactic acid block copolymers, and combinations thereof.

In some embodiments, the polymers used in accordance with the present invention are selected to have a thermal transition within the physiologically relevant temperature range, relevant to OVDs. The OVDs may include any thermo-responsive polymer such as poly(ethylene oxide)-poly(propylene oxide)-poly(ethylene oxide) (PEO-PPO-PEO) triblocks, random or alternating reverse thermo-responsive PEO-PPO block copolymers such as those described in U.S. Patent Application No. 10/211,228 and other polymers comprising PEO and PPO segments. Also relevant are N-alkyl substituted acrylamides (e.g., poly-N-isopropyl acrylamide [PNIPAAm]), cellulose derivatives (e.g., hydroxypropyl methylcellulose and hydroxypropyl cellulose), alternating or random, and various amphiphilic polymers such as poly(ethylene oxide)-polylactic acid block copolymers, and combinations thereof, among other RTG-displaying polymeric system.

In some embodiments, the OVD comprises poly(N-isopropyl acrylamide) (PNIPAAm).

In other embodiments, the OVD comprises at least one polymer selected amongst polymers belonging to the family of poly(ethylene oxide)/poly(propylene oxide)/poly(ethylene oxide) (PEO-PPO-PEO) triblocks, commercially available as Pluronic.

In further embodiments, the OVD comprises {EO}₉₉-{PO}₆₇-{EO}₉₉ triblock, known as Pluronic F127.

In some embodiments, the OVD comprises a tetrafunctional block polymers of polyoxyethylene and polyoxypropylene condensed with ethylenediamine (commercially available as Tetronic).

Based on the selection of polymers for making the OVDs of the invention, the OVDs may be deployed in the eye below their thermal transition, where they exhibit low viscosity, making injecting them to the eye easy and simple. Upon contact with the tissues of the eye, due to their higher temperature, at typically 34°C, the low viscosity solution suitably gels, to generate a protective shield for the corneal endothelial cells. Due to the versatility of the polymers utilized in OVDs of this invention, the gels formed may be tailored and fine-tuned over a broad range of rheological and mechanical properties.

The key feature of these OVDs is the creation of a stimulus-responsive device that makes its deployment, performance and removal safe and efficacious. The thermo-responsive component(s) may be applied in one shot, simultaneously, or one after another, in cases the OVD composition comprises more than one thermo-responsive component. Their components may have the same composition and/or concentration, and they may also be responsive not only to one but to two or more different environmental stimuli or may perform other tasks, such as, without limitation, other chemical, physical, mechanical or biological functions. The composition and properties of the thermo-responsive component/s may also vary both in space and in time, along all directions, isotropically or anisotropically, at different points of the OVD.

In other embodiments, the thermo-responsive component(s) may be "programmed" so that their viscosity changes over time. It is also an object of the invention to generate solid particles of environmentally responsive component(s), including, without limitation, thermo-responsive component(s).

The different components of the OVD, polymeric or not, as well as different parts of the OVD may be deployed simultaneously or one after another.

In some embodiments, the OVD is a copolymer of Pluronic F127 and L101 (PF228).

In some embodiments, the ratio between F127 and L101 is 50:50.

In some embodiments, the OVD is a copolymer of F127 and P103 (PF230).

In some embodiments, the ratio between F127 and P103 is selected from 10:90, 37:63 and 86:14.

In some embodiments, the OVD is a copolymer F127 and P123 (PF250).

In some embodiments, the ratio between F127 and P123 is 50:50.

The invention also discloses a multi-component OVD that can be made to disappear by lowering the temperature and simply liquefying, partially or fully, one or more of the thermo-responsive component/s of the OVD.

The OVD may comprise components (additives, e.g., non-therapeutic additives) that respond to different stimuli, for the purpose of deploying and/or removing the OVD and/or, or during its performance during the surgical procedure and/or for any other purpose. These additional purposes may be, without limitation, chemical, physical, mechanical, biological or combinations thereof.

The final product may consist, partially or totally, of blends, semi-IPNs, IPNs or copolymers, including either all or part of the thermo-responsive component/s, in any combination. The final OVD may be homogeneous in space or may consist of different zones displaying different properties, these zones being able to be nanometric, up to macroscopic, continuous or discontinuous, creating independent or interconnected domains within the system, having several geometries, architectures and spatial arrays, dispersed homogeneously or heterogeneously, isotropically or anisotropically.

In some embodiments, the OVD comprises one or more environmentally responsive component(s), such as, among others, and without limitation, thermo-responsive or pH-sensitive agents that respond to other environmental stimuli, such as, among others, and without limitation, ionic strength, light, electrical and/or magnetic fields, and combinations thereof.

In other embodiments, the OVDs may be partially or totally biodegradable. It is another embodiment of this invention that said biodegradability may be confined in time and in space. Biocompatibility and sterilizability are two additional important attributes of the OVDs disclosed herein. Furthermore, the materials as well as their degradation products are non-toxic and, therefore, can be used both on the surface of the body as well as internally.

In some embodiments, the OVD polymers are selected to display temperature-dependent coiling-uncoiling ability in aqueous media. Thermo-responsive chains may be covalently grafted onto the surface or attached in any other way, such as, and without limitation, by generating hydrogen bonds, ionic bonds or by mechanical interlocking with the substrate.

It is also an object of the compositions of the present invention to include also additional materials that fulfill other roles, including, without limitation, rendering the OVD with the desired mechanical or rheological behavior or with the appropriate transport properties or with the suitable attachment behavior to the tissue or organs or any other desirable and advantageous chemical, physical or biological characteristics, and combinations thereof.

In some embodiments, the OVD of the invention comprises at least one additive as disclosed herein.

In some embodiments, the OVD of the invention consists of one or more polymeric materials as disclosed herein.

It is an object of this invention to engineer OVDs that consist of one or more layers. In some embodiments, a tissue facing layer is formed. In other embodiments, said tissue facing layer is aimed at a two-fold objective: maximizing its tight attachment to the site, following the deployment stage and, and minimizing its attachment to the site and allowing its removal to be minimally disruptive to the tissue, when required.

In some embodiments, the OVD allows direct visualization of the tissues or organs the OVD is in contact with and beneath it, through the OVD, at all times during the procedure.

In some embodiments, the OVD comprises additives in particulate form. In some embodiments, the particles are nanometric. In other embodiments, the solid particles dissolve or degrade over time, contributing to the performance of the OVD.

In some embodiments, the OVD consists of RTG-displaying polymer(s). In some embodiments, the OVD consist of more than one RTG-displaying polymer, and each may be liquefied sequentially, as a form of removing the OVD from the site.

In other embodiments, the OVDs disclosed comprise a component that renders the surface layer with desirable properties, such as, without limitation, rendering the surface with adhesive or anti-adhesive properties.

It should be stressed that throughout this document the terms "reverse thermo-responsive polymer" and "thermo-responsive polymer" will be used interchangeably and they will mean a "reverse thermo-responsive polymer".

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig.** 1 shows layered reverse thermo-responsive structures.
**Figs. 2A-B** show layered reverse thermo-responsive structures.
**Fig. 3** shows storage modulus of the PF solution at 34°C compared to commercial OVD.
**Fig. 4** shows commercial OVDs refractive index compeer to PF solutions.
**Fig. 5** shows stained reaction with Fluorescein isothiocyanate.
**Figs. 6A-B** show Healthy corneal endothelial cells (**6A**) Corneal endothelial cells after trauma (**6B**).
**Fig. 7** shows endothelial cell layer (**left**) after using PF solution and (**right**) after using Biolon solution.
**Fig. 8** shows G' of the competing materials, commercially available.
**Figs. 9A-B** show RTG behavior of PF250 50:50 15% wt. at 5-37°C (**9A**); transparency test of the PF250 50:50 15% wt. gel (**9B**).
**Fig. 10** shows RTG (reverse thermo-responsive) behavior of PF in water 25% wt with different DP.
**Fig. 11** shows RTG behavior of PF-3.6 in water 5-25% wt.
**Fig. 12** shows RTG behavior of F127:P103 86:14 at 5-37°C.
**Fig. 13** shows RTG behavior of F127:P103 68:32 at 5-37°C.
**Fig. 14** shows RTG behavior of F127:P103 37:63 at 5-37°C.

### DETAILED DESCRIPTION OF THE INVENTION

Hereinbelow, follow examples that illustrate the invention disclosed herein. The inventors have chosen to confine themselves to the simplest OVDs, even though more complex compositions are contemplated. Complex OVDs have been prepared in accordance with the invention but respective data is not provided.

Furthermore, for the sake of clarity, conciseness and simplicity, and without limiting the scope of the invention in any form or fashion, the inventors have chosen to illustrate the invention hereby disclosed, by focusing on OVDs comprising reverse thermo-responsive polymers, and describing OVDs comprising one reverse thermo-responsive polymer. Additional OVDs have been prepared in accordance with the invention but respective data is not provided.

Moreover, for the sake of clarity, conciseness and simplicity, and without limiting the scope of the invention in any form or fashion, the inventors have chosen to illustrate the invention by employing thermo-responsive polymers comprising poly(ethylene oxide) and poly (propylene oxide) blocks, even though the compositions of the present invention include all families of thermo-responsive polymers and also may be engineered to respond to other environmental stimuli and combinations thereof. Focusing and exemplifying the invention using one particular family of thermo-responsive polymers should not be construed as limiting the scope of this invention.

Some of the embodiments of the invention disclosed are exemplified using a family of polymers comprising poly(ethylene oxide) (PEO) and poly(propylene oxide) (PPO) chains. These may be part of diblocks such as poly(ethylene oxide)-poly(propylene oxide) molecules or triblocks such as poly(ethylene oxide)-poly(propylene oxide)-poly(ethylene oxide) (PEO-PPO-PEO) triblocks, commercially available as Pluronic©, and primarily, {EO}₉₉-{PO}₆₇-{EO}₉₉, known as Pluronic F127. Also, high molecular weight reverse thermo-responsive polymers produced by covalently binding PEO-PPO-PEO triblocks using reactive bifunctional molecules such as disiocyanates, diacylchlorides and phosgene, among others, were used as well. Among them, hexamethylene diisocyanate (HDI) was typically used. Additionally, block polymers consisting of poly(ethylene oxide) (PEO) and poly(propylene oxide) (PPO) segments, coupled *via* diverse coupling agents, such as disiocyanates, diacylchlorides, phosgene, among others, were used as well.

### [a] Synthesis of PF-127.

Pluronic F-127 (molecular weight 12,600) was poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different F-127/ HDI ratio resulted in different degree of polymerization (DP).

### [b] Synthesis of F127 combined with PPG.

Pluronic F-127 (molecular weight 12,600) and PPG (various molecular weights) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:PPG)/ HDI ratio resulted in different degree of polymerization (DP).

### [c] Synthesis of F127 combined with PEG.

Pluronic F-127 (molecular weight 12,600) and PEG (various molecular weights) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:PEG)/ HDI ratio resulted in different degree of polymerization (DP).

### [d] Synthesis of PF-228.

Pluronic F-127 (molecular weight 12,600) and Pluronic L-101 (molecular weight 4,950) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:L-101)/ HDI ratio resulted in different degree of polymerization (DP).

### [e] Synthesis of PF-230.

Pluronic F-127 (molecular weight 12,600) and Pluronic P-103 (molecular weight 5,750) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:P-103)/ HDI ratio resulted in different degree of polymerization (DP).

### [f] Synthesis of PF-248.

Pluronic F-127 (molecular weight 12,600) and Pluronic L-121 (molecular weight 5,750) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:L-121)/ HDI ratio resulted in different degree of polymerization (DP).

### [g] Synthesis of PF-250.

Pluronic F-127 (molecular weight 12,600) and Pluronic P-123 (molecular weight 5,750) were poured in a three-necked flask and dried. Then, the corresponding amount of HDI and SnOct₂ (0.64 wt %) were added to the reaction mixture and reacted at 80°C for 30 minutes under mechanical stirring (160 RPM) and dry nitrogen atmosphere. The polymer produced was dissolved in chloroform and precipitated in a petroleum ether 40-60/ ethyl ether mixture (1:1). Finally, the polymer was washed repeatedly with portions of petroleum ether and dried. Different (F-127:P-123)/ HDI ratio resulted in different degree of polymerization (DP).

### [h] Synthesis of Poy(ether-carbonate)s.

These polymers were synthesized by copolymerizing poly(ethylene glycol) and poly(propylene glycol) segments utilizing phosgene as the coupling molecule. The different reactivity of phosgene's two functionalities allowed binding the two constituents, in both an alternate or random mode. The synthesis of the alternating poly(ether-carbonate)s was carried out following a two-step reaction, as known in the art. The first step was the PEO dichloroformate synthesis, followed by the reaction between the PEO derivative and the PPG chain, to produce the final block copolymer. The random poy(ether-carbonate)s were synthesized by a similar one-pot reaction, as described elsewhere in detail.

### [i] Synthesis of Poy(ether-ester)s.

The synthesis is exemplified hereby for a copolymer containing PEO6000 and PPO3000 segments. Equimolar amounts of dry PEG6000 and dry PPG3000 were dissolved in 30 ml dry chloroform in a 250 ml flask. Triethylamine (2:1 molar ratio to PEG) was added to the reaction mixture, followed by the dropwise addition of the diacyl chloride (2:1 molar ratio to PEG) in dry chloroform over a period of 30 minutes at 40°C, under magnetic stirring. Then, the temperature was risen to 60°C and the reaction was continued for additional 90 minutes. The polymer produced was separated from the reaction mixture by adding to it about 600 ml petroleum ether 40-60. The lower phase of the two-phase system produced was separated and dried at RT. Finally, the polymer was thoroughly washed with petroleum ether and dried. Light yellow, brittle and water soluble powders were obtained.

### [j] Synthesis of Poy(ether-ester-carbonate)s.

The synthesis is exemplified hereby for a copolymer containing PEO6000 and PPO3000 segments, caprolactone blocks comprising four repeating units and phosgene. The (CL)₄-PEO6000-(CL)₄ triblock was synthesized as follows: 30.3 g of PEG6000 were dried at 120°C under vacuum for 2 hours. Then, 10.1 g ε-caprolactone and 0.05 g stannous 2-ethyl-hexanoate were added. The reaction mixture was heated at 145°C for 2.5 hours in a dry nitrogen atmosphere. Finally, the reaction mixture was cooled to room temperature, dissolved in chloroform, precipitated in petroleum ether and dried at room temperature. Once the (CL)₄-PEO6000-(CL)₄ triblock was obtained, the reaction with phosgene and the final reaction with the PPG chain were performed as described above.

In some instances biologically active molecules were added to the systems, by blending them into one or more components of the OVD. In other instances, the active molecules were covalently bound to any of the components of the OVD, *via* a biodegradable or a non- biodegradable coupling molecule.

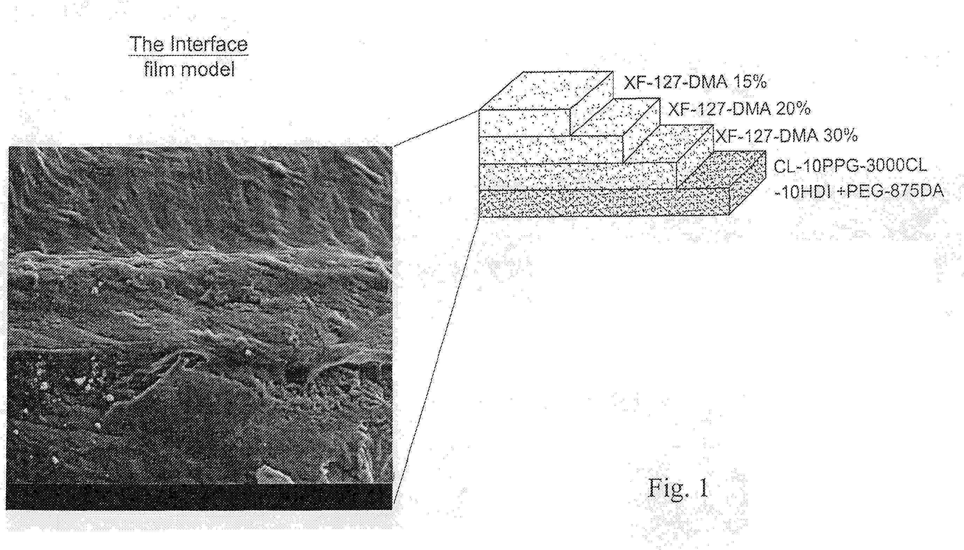

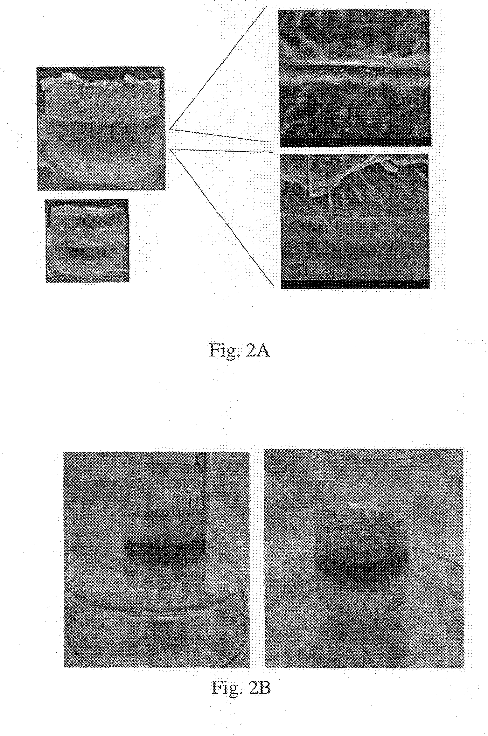

In some embodiments, a concentration gradient was engineered into the OVD, by varying various experimental and compositional parameters. The objective was to combine sturdiness and maximum protection of the corneal endothelial cells, with enhanced chain mobility at the very interface between the OVD and the corneal endothelium. **Figs. 1** and **2** illustrate this embodiment, comprising layers that differ in the concentration of the RTG-displaying polymer. The SEM micrograph in **Fig. 2A** (above and below) show the tight interface formed between the different layers.

**Fig. 2A-B** show a tri-layered thermo-responsive gel, colored for visualization purposes.

In addition to one or more RTG-displaying components, the OVDs of the present invention may consist of two or more biodegradable components having different rates of degradation.

In some embodiments, in addition to one or more RTG-displaying components, the OVDs may comprise also a water soluble polymer that does not display RTG behavior.

### Rheological analysis

The rheological analysis was conducted using a parallel plate HAAKE RheoScope-1 Optical rheometer. First, G' values attained by various solutions of relatively high concentrations at 37°, were measured. **Table 1** shows the difference between various solutions and commercial products based on Hyaluronic acid (HA).

**Table 1. Difference between various solutions and commercial products based on Hyaluronic acid (HA). Biolon and Viscoat are based on Sodium hyaluronate.**

| Sample | DP | Concentration [% w/w] | G' (37°C) [Pa] |
|---|---|---|---|
| PF127 | 4.3 | 9% | 1,482 |
| PF127 | 4.3 | 12% | 5,522 |
| PF127 | 4.3 | 15% | 10,690 |
| BIOLON | | 1% | 115 |
| VISCOAT | | 1% | 290 |

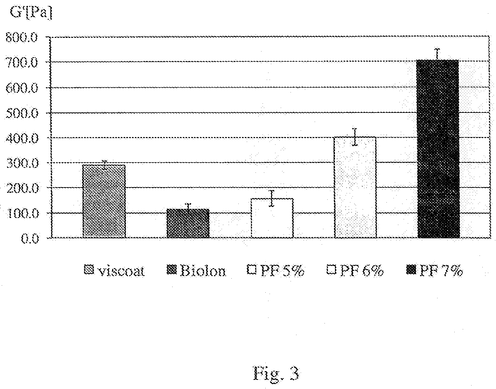

More diluted PF solutions exhibiting lower G' values (at 34 °C), were compared to commercially available solutions. G' increased with concentration, shifting from around 160 Pa from a 5 wt% gel, up to above 700 Pa for a 7 wt% concentration, in the case of a polymer with DP=4.0. Several of the OVDs of this invention, displayed at low temperatures lower G' values than the those of OVDs being currently used in the clinic, on one hand, and higher than the G' values exhibited by OVDs presently in clinical use, on the other hand (**Fig. 3**). These data, clearly illustrate the unique and highly advantageous ability of the novel OVDs disclosed herein. More specifically, the OVDs taught by the present invention combine low viscosity at the deployment and removal stages, so they can easily be deployed and fully and rapidly removed from the eye, and higher viscosity and enhanced robustness when required to protect the corneal endothelium.

### Gel Transparency

During cataract surgery, the OVD is injected into the capsular bag before the removal of the cataractous lens and it cannot reduce the surgeon's completely clear view of the eye. Consequently, the gel must be fully transparent, with a refractive index similar to that of the biological medium inside the capsular bag.

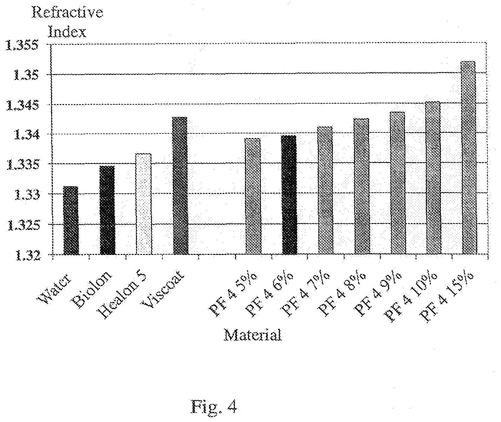

The refractive index of several OVDs of the present invention were measured and compared to that of the biological medium. Also, the turbidity and the transparency of the OVDs was measured using an UV spectrophotometer (**Fig. 4**).

### Rheological behavior

The handling and user friendly characteristics of the OVDs of this invention were also assessed. First, the injectability was measured and compared to commercial products based on HA (Biolon, Healon5, Viscoat). Additionally, an artificial capsular bag model was built to test the mobility of the gel during and after the implantation at 34°C, inside a closed cavity.

The injectability test was designed to answer two main questions: [i] what is the gelation time of the PF solution and [ii] how does the PF solution flow through a 27G canulla.

Three PF (DP=4.0) polymer solutions were evaluated (5%, 6%, and 7%). **Table 2** below shows that the gelation time decreased, as the gel concentration increased. 0.1 ml of the PF solution was placed on a vertically positioned hand palm at 34°C (to mimic the temperature in the eye) to measure the gelation time and the distance (in centimeters) the solution will cover before gelling, under those conditions (**Table 2**).

**Table 2. gelation time versus gel concentration.**

| | Gelation Time [sec] | Flow Distance [mm] | Temperature [°C] |
|---|---|---|---|
| PF 5% | 6.1 | 34 | 34 |
| PF 6% | 4.2 | 30 | 34 |
| PF 7% | 3.0 | 27 | 34 |

As mentioned above, the PF solution must be deliverable through a 27G cannula. All solutions were tested at two temperatures, 10°C and 21°C, the latter being the operating room (OR) temperature. The low concentration solutions (5%, 6% and 7%) were easily syringable through 27G cannulae at both temperatures.

To mimic the capsular bag, a model capsular bag was prepared using Polyethylene film. The size of the artificial capsular bag was 2x2 cm. 1.0 ml of the polymer solution was injected into the polymeric bag at 21°C followed by 0.1 ml of air. For each polymer, six plastic bags were prepared, three were placed in a water bath at 34°C for 1 min and the other rest were kept at OR temperature as a control group. After 1 min at 34°C we tracked after the air bubble movement in the gel. In all the gels, the air bubble exhibited the same velocity, save for the low concentration gel (5%), which was even quicker. Finally, a 27G cannula was inserted into the plastic bag and moved from side to side to purposely try to damage the gel at physiological temperature. Neither the bubble test nor the cannula abrasion affected the gel.

### Removal Time and Endothelial Cell Protection

A common complication of OVDs after cataract surgery is an increase in postoperative intraocular pressure (IOP) because of a fraction of the OVD remained in the anterior chamber and obstructed the trabecular meshwork. In modern small incision cataract surgery, the incision sizes are usually between 3.0 to 4.0 mm. Therefore, the OVD is not easily and fully washed out by irrigation. In a standardized laboratory setup, we used porcine eyes to evaluate the removal of a Poly(F127)-based OVD from a capsular bag. For better visualization, the polymer was stained by covalently binding Fluorescein isothiocyanate (FITC) to it, as show in the figure below.

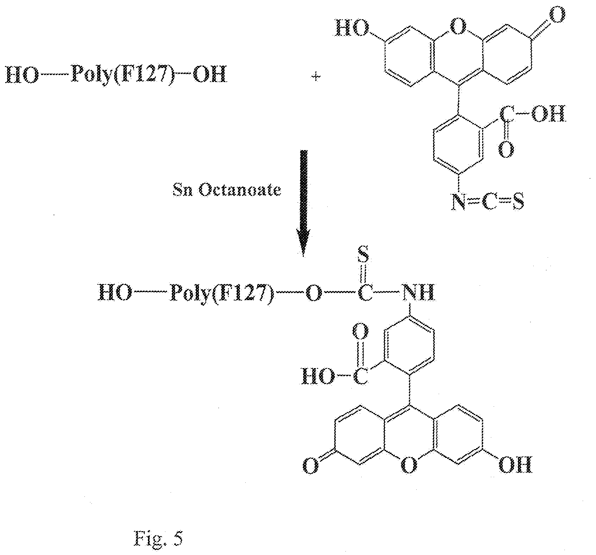

Tagged PF was used in six fresh porcine eyes, to evaluate the removal and six fresh porcine eyes were used with PF solution without the fluorescein marker. The eyes were pre-conditioned at 34°C to simulate the physiological conditions and PF was used as the OVD material. In the last step of the procedure, BSS solution at 20°C was injected into the capsular bag, to remove the PF by liquefying the gel. **Fig. 5** shows the model eye before (**right**) and after (**left**) the gel removal, while the eyes were radiated using a UV lamp at 365 nm. In the left figure, FITC solution was injected at the limbal corneal interface, to mark the corneal site that does not glow, after removal of the gel from the capsular bag. After the polymer was completely removed, the cornea was dissected *via* a limbal incision. The corneas were inspected microscopically using a fluorescein lamp, to show that the stain was removed from the eye with no residual fluorescence being detectable.

The corneal endothelium consists of a cellular monolayer on the posterior corneal surface that had no regenerative ability after injury. Intraocular manipulation, such as that conducted during phacoemulsification, generated fluid, lens fragment turbulence and the surgical equipment could lead to endothelial cell damage.

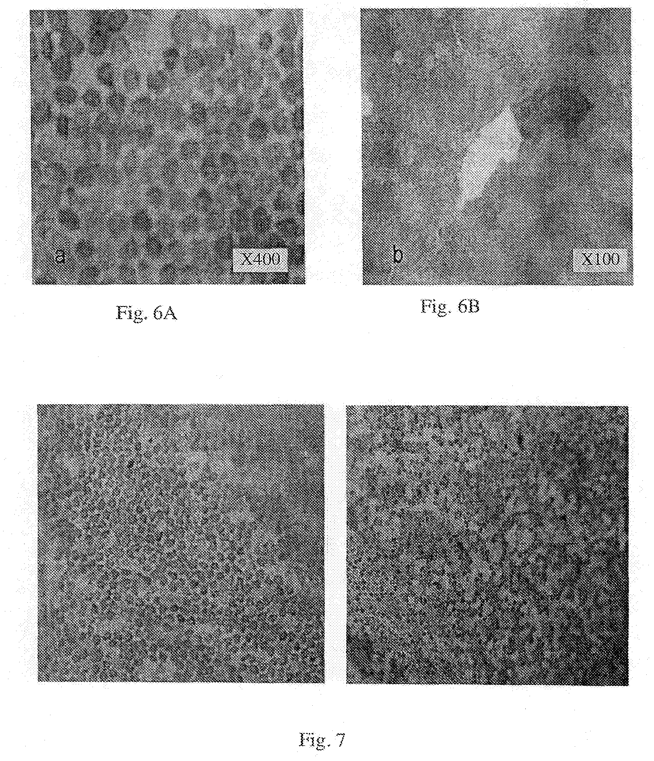

**Fig. 6****.** (left) shows healthy corneal endothelial cells with hemocolor stain, while **Fig. 6** (right) shows corneal endothelial cells after cannula-caused trauma during cataract surgery. The latter exemplifies the type of damage corneal endothelium often suffers during cataract surgery.

During the development of polymers for use as OVDs, one of the critical challenges was to avoid the OVD itself detrimentally affecting the endothelial cells. To assess this important concern, PF and several commercial OVDs were put in contact with corneal endothelium and their effect on the cells was recorded. Each OVD was used in four fresh porcine eyes. After the OVDs were removed, a dissection of the cornea was done by limbal incision. The corneas were examined by light microscopy, to determine the extent of endothelial cell damage caused by each of the OVDs studied. **Fig. 7** show corneal endothelium after being in contact with a PF (left) and Biolon (right). It is apparent from the photos that there was no large difference between the two OVDs, and if something, PF proved to be less disruptive to the cells than Biolon.

### Evaluation of the viscosity of the OVD polymers

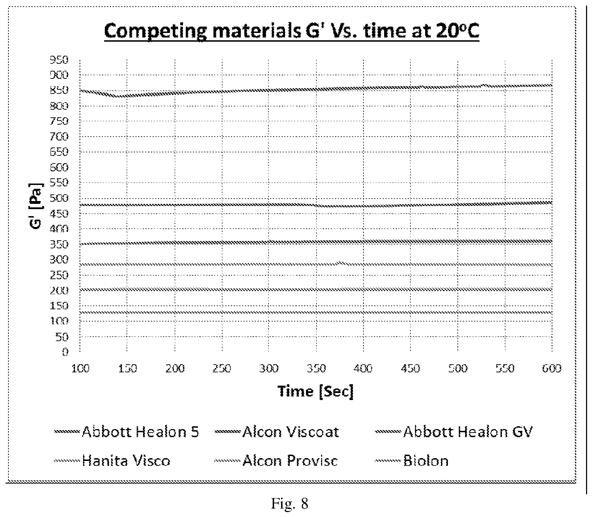

In order to evaluate the viscosity of the polymers of the present invention, the OVDs of the present invention were compared to the OVD products on the market. A rheological test comparing them was conducted, as shown in **Fig. 8**.

**Table 3** show the G' values of several commercially available OVD products and some of the OVD systems describes herein. The table also present the ratios of the G' values of different products, when ratioed to the G' value of Healon 5 (Abbott), the most viscous commercial OVD tested, and to Biolon (BTG), the less viscous commercial OVD tested.

As can be seen, the OVD systems of the present invention successfully combined very low viscosities at a low temperature and much higher viscosities at a higher temperature. So much so, that, for example, at 12°C, a 20% OVD consisting of F127:P103 at a 86:14 ratio, was seven times less viscous than the less viscous commercially available OVD tested (Biolon), while at 22°C, the same OVD became 16 time more viscous than the most viscous commercially available OVD tested.

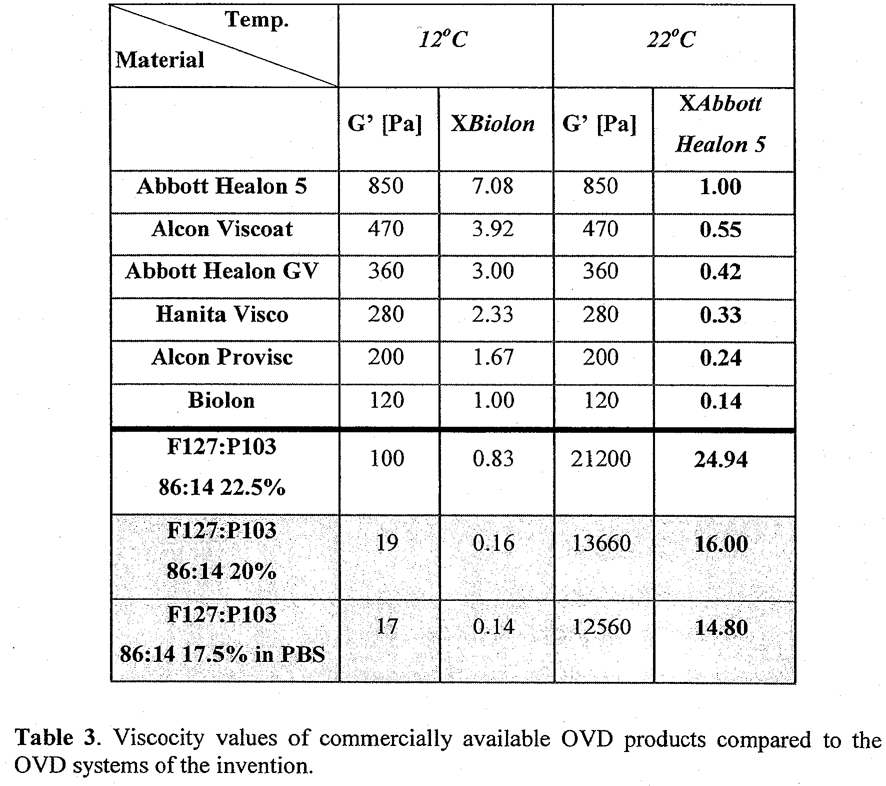

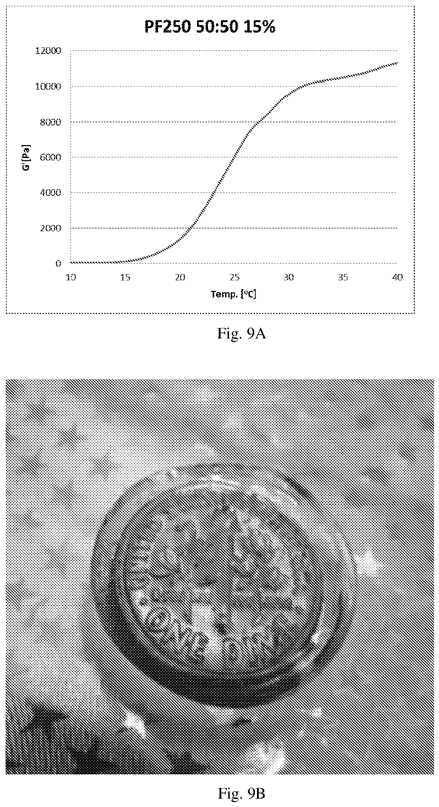

An evaluation of the transperancy of PF250 50:50 15% (see its rheological behavior in the **Fig. 9A**), is shown in the OVD covered dime coin (**Fig. 9B**).

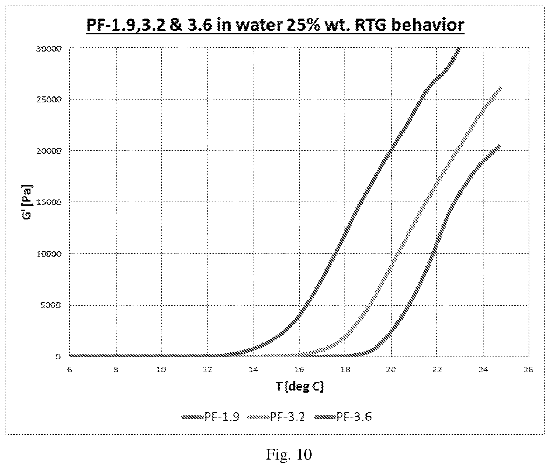

**Fig. 10** shows RTG (reverse thermo-responsive) behavior of PF in water, 25% wt with different DP.

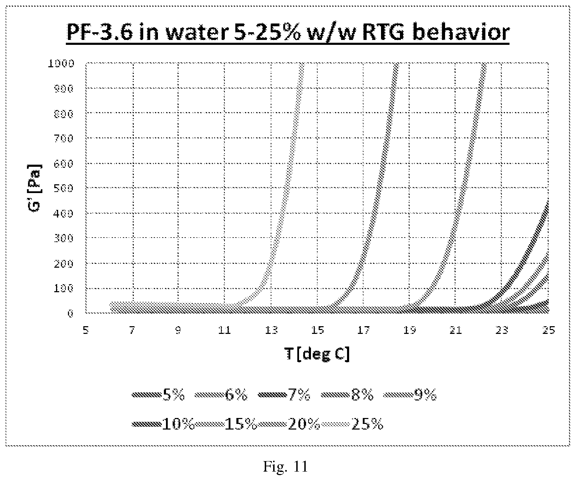

**Fig. 11** shows RTG behavior of PF-3.6 in water, 5-25% wt.

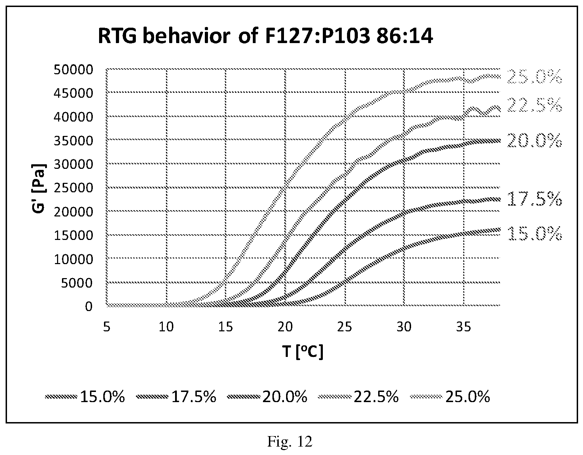

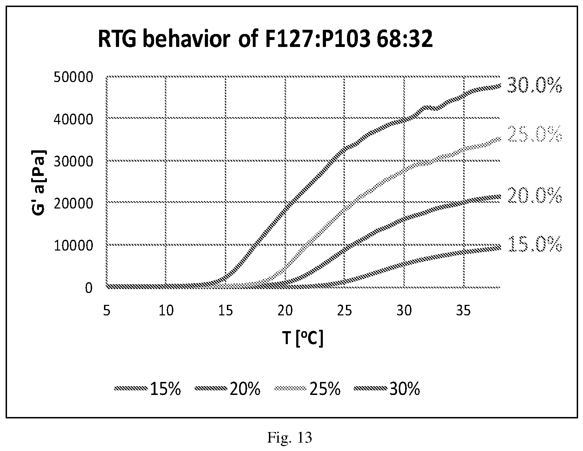

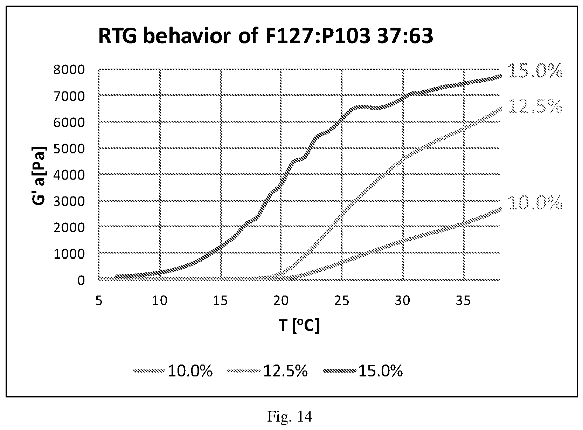

**Figs. 12** through **14** present the results of experiments in which RTG behavior of various PFs was measured such that solutions of the polymers in water in different concentrations were prepared, and the rheological behavior was measured as described above.

## Claims

1. An ophthalmic viscosurgical device (OVD) **characterized by** comprising a reverse thermo-sensitive material.

2. The OVD of claim 1, for use in a method of treatment of an eye of a subject.

3. The OVD according to any one of claims 1 and 2, being formed of a single compound, or a mixture of compounds or a combination of several compounds.

4. The OVD according to claim 3, comprising the single compound, or the mixture of compounds or the combination of several compounds in a solution.

5. The OVD according to any one of claims 1 to 4, wherein said material is a polymer.

6. The OVD according to claim 5, wherein said reverse thermo-sensitive polymer is in the form of hydrogel.

7. The OVD according to any one of claims 5 and 6, wherein the polymer is selected from N-alkyl substituted polyacrylamides, cellulose derivatives, amphiphilic polymers, poly(ethylene oxide)-polylactic acid block copolymers, poly(ethylene oxide)-poly(propylene oxide)-poly(ethylene oxide) (PEO-PPO-PEO) triblocks, random or alternating reverse thermo-responsive PEO-PPO block copolymers, copolymers comprising PEO and PPO segments, and any combination thereof.

8. The OVD according to claim 7, wherein the polymer is poly(N-isopropyl acrylamide) (PNIPAAm).

9. The OVD according to claim 7, wherein the polymer is {EO}₉₉-{PO}₆₇-{EO}₉₉ triblock (Pluronic F127).

10. The OVD according to claim 7, being selected from copolymer of Pluronic F127 and L101 (PF228), a copolymer of F127 and P103 (PF230) and a copolymer of F127 and P123 (PF250).

11. The OVD according to any one of the preceding claims, further comprising one or more environmentally responsive component(s) that respond to one or more environmental stimuli selected from ionic strength, irradiation, electrical and/or magnetic fields, and combinations thereof.

12. The OVD according to claim 11, being partially or totally biodegradable.

13. The OVD according to any one of claims 1 to 12, being in the form of a multilayer or a multi-zone structure.

14. The OVD according to any one of claims 1 to 13, for use in one or more of: maintaining anterior chamber space in the eye, protecting cornea iris and other eye structure during phacoemulsification, segregating damaged iris from phacoemulsification, creating a stable yet flexible environment for eye surgery, and in maintenance of the anterior chamber after phaco-trabecalectomy or trabecalectomy alone.

15. The OVD according to any one of claims 1 to 14, for use during corneal transplant, or during anterior chamber intraocular lens implantation.

## Patentansprüche

1. Ophthalmische viskochirurgische Vorrichtung (OVD), **dadurch gekennzeichnet, dass** sie ein reverses wärmeempfindliches Material umfasst.

2. OVD nach Anspruch 1 zur Verwendung in einem Verfahren zur Behandlung eines Auges eines Subjekts.

3. OVD nach einem der Ansprüche 1 und 2, die aus einer Einzelverbindung oder einem Gemisch von Verbindungen oder einer Kombination mehrerer Verbindungen gebildet ist.

4. OVD nach Anspruch 3, umfassend die Einzelverbindung oder das Gemisch von Verbindungen oder die Kombination mehrerer Verbindungen in einer Lösung.

5. OVD nach einem der Ansprüche 1 bis 4, wobei das Material ein Polymer ist.

6. OVD nach Anspruch 5, wobei das reverse wärmeempfindliche Polymer in der Form von Hydrogel vorliegt.

7. OVD nach einem der Ansprüche 5 und 6, wobei das Polymer ausgewählt ist aus N-alkylsubstituierten Polyacrylamiden, Zellulosederivaten, amphiphilen Polymeren, Poly(ethylenoxid)-polymilchsäure-Blockcopolymeren, Poly(ethylenoxid)poly(propylenoxid)-poly(ethylenoxid) (PEO-PPO-PEO) Triblöcken, statistischen oder alternierenden reversen temperaturresponsiven PEO-PPO-Blockcopolymeren, Copolymeren, die PEO- und PPO-Segmente umfassen, und jeder beliebigen Kombination davon.

8. OVD nach Anspruch 7, wobei das Polymer Poly(N-isopropylacrylamid) (PNIPAAm) ist.

9. OVD nach Anspruch 7, wobei das Polymer {EO}₉⁹-{PO}₆₇-{EO}₉₉ Triblock (Pluronic F127) ist.

10. OVD nach Anspruch 7, ausgewählt aus einem Copolymer aus Pluronic F127 und L101 (PF228), einem Copolymer aus F127 und P103 (PF230) und einem Copolymer aus F127 und P123 (PF250).

11. OVD nach einem der vorhergehenden Ansprüche, ferner umfassend eine oder mehrere auf Umwelteinflüsse reagierende Komponente(n), die auf einen oder mehrere Umweltreize reagieren, ausgewählt aus lonenstärke, Bestrahlung, elektrischen und/oder magnetischen Feldern und Kombinationen davon.

12. OVD nach Anspruch 11, die teilweise oder vollständig biologisch abbaubar ist.

13. OVD nach einem der Ansprüche 1 bis 12, die in der Form einer Mehrschicht- oder einer Mehrzonenstruktur vorliegt.

14. OVD nach einem der Ansprüche 1 bis 13 zur Verwendung in einem oder mehreren der folgenden Bereiche: Aufrechterhaltung des Vorderkammerraums im Auge, Schutz der Kornea, Iris und anderer Augenstruktur während der Phakoemulsifikation, Trennen der geschädigten Iris von der Phakoemulsifikation, Schaffen einer stabilen und dennoch flexiblen Umgebung für Augenchirurgie und bei Erhaltung der Vorderkammer nach Phako Trabekulektomie oder Trabekulektomie allein.

15. OVD nach einem der Ansprüche 1 bis 14 zur Verwendung während kornealer Transplantation oder während der Implantation von intraokularer Vorderkammerlinse.

## Revendications

1. Dispositif viscochirurgical ophtalmique (OVD) **caractérisé en ce qu'**il comprend un matériau thermosensible réversible réciproquement.

2. OVD de la revendication 1, pour utilisation dans un procédé de traitement de l'oeil d'un sujet.

3. OVD selon l'une quelconque des revendications 1 et 2, étant formé d'un seul composé, ou d'un mélange de composés ou d'une combinaison de plusieurs composés.

4. OVD selon la revendication 3, comprenant le seul composé, ou le mélange de composés ou la combinaison de plusieurs composés dans une solution.

5. OVD selon l'une quelconque des revendications 1 à 4, dans lequel ledit matériau est un polymère.

6. OVD selon la revendication 5, dans lequel ledit polymère thermosensible réversible réciproquement se présente sous forme d'un hydrogel.

7. OVD selon l'une quelconque des revendications 5 et 6, dans lequel le polymère est choisi entre des polyacrylamides substitués par un groupe N-alkyle, des dérivés cellulosiques, des polymères amphiphiles, des copolymères séquencés de poly(oxyde d'éthylène)-acide polylactique, triséquencés de poly(oxyde d'éthylène)-poly(oxyde de propylène)-poly(oxyde d'éthylène) (PEO-PPO-PEO), des copolymères séquencés de PEO-PPO thermosensibles réversibles réciproquement aléatoires ou alternés, des copolymères comprenant des segments PEO et PPO, et toute combinaison de ceux-ci.

8. OVD selon la revendication 7, dans lequel le polymère est le poly(N-isopropylacrylamide) (PNIPAAm).

9. OVD selon la revendication 7, dans lequel le polymère est un triséquencé de {EO}₉₉-{PO}₆₇-{EO}₉₉ (Pluronic F127) .

10. OVD selon la revendication 7, étant choisi entre un copolymère de Pluronic F127 et L101 (PF228), un copolymère de F127 et P103 (PF230) et un copolymère de F127 et P123 (PF250).

11. OVD selon l'une quelconque des revendications précédentes, comprenant en outre un ou plusieurs composants réagissant à l'environnement qui répondent à un ou plusieurs stimuli environnementaux choisi entre la force ionique, l'irradiation, des champs électriques et/ou magnétiques, et leurs combinaisons.

12. OVD selon la revendication 11, étant partiellement ou totalement biodégradable.

13. OVD selon l'une quelconque des revendications 1 à 12, se présentant sous la forme d'une multicouche ou d'une structure multizone.

14. OVD selon l'une quelconque des revendications 1 à 13, pour utilisation dans un ou plusieurs parmi : le maintien d'un espace de la chambre antérieure dans l'oeil, la protection de la cornée, de l'iris et d'une autre structure de l'oeil pendant une phacoémulsification, la séparation de l'iris endommagé de la phacoémulsification, la création d'un environnement à la fois stable et souple pour une chirurgie oculaire, et lors du maintien de la chambre antérieure après une phaco-trabéculectomie ou une trabéculectomie seule.

15. OVD selon l'une quelconque des revendications 1 à 14, pour utilisation lors d'une greffe de la cornée ou lors d'une implantation de lentille intraoculaire à chambre antérieure.
